# EUROPÄISCHE PATENTSCHRIFT

(11) **EP 2 926 799 B1**
(45) Veröffentlichungstag und Bekanntmachung des Hinweises auf die Patenterteilung: **30.09.2020**
(21) Anmeldenummer: 15160703.3
(22) Anmeldetag: 25.03.2015
(51) Int. Cl.: A61K 8/34, A61Q 17/04, A61Q 19/00, A61K 8/81

(54) **KOSMETISCHE ZUBEREITUNG MIT ERHÖHTER HAUTBEFEUCHTUNG**
COSMETIC PREPARATION WITH INCREASED SKIN HYDRATION
PRÉPARATION COSMÉTIQUE POUR HYDRATATION ACCRUE DE LA PEAU

(30) Priorität: 01.04.2014 DE 102014206218
(43) Veröffentlichungstag der Anmeldung: 07.10.2015
(73) Patentinhaber: Beiersdorf AG, 20253 Hamburg (DE)
(72) Erfinder: Bleckmann, Andreas, 22926 Ahrensburg (DE); Lerg, Heike, 22303 Hamburg (DE); Schlenker, David, 22607 Hamburg (DE)

(56) Entgegenhaltungen:
- EP-A2- 2 243 463
- WO-A1-03/059311
- WO-A2-2009/135755
- WO-A2-2013/064391
- ANONYMOUS: "Discover the latest ingredients, formulations and the most innovative beauty products", INNOVATION ZONE 2012 IN-COSMETICS ASIA, 1. November 2012 (2012-11-01), XP055206294,

## Beschreibung

Die vorliegende Erfindung betrifft eine kosmetische Zubereitung enthaltend ein Acrylat-Copolymer mit einer Glasübergangstemperatur von -5 bis -15 C°, gemessen mittels DSC und Glycerin, die frei ist von partikulären Eisenoxiden.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Die Vielzahl an kommerziell erhältlichen Hautpflegeprodukte darf jedoch nicht darüber hinwegtäuschen, dass diese Zubereitungen des Standes der Technik eine Reihe von Nachteilen aufweisen.

Eine wesentliche Aufgabe von Hautpflegemitteln besteht darin, die Haut über einen längeren Zeitraum hin zu befeuchten bzw. feucht zu halten. Ein typisches, viel verwendetes Hautbefeuchtungsmittel ist Glycerin. Glycerin hat jedoch den Nachteil, erst in höheren Mengen wirksam hautbefeuchtend zu sein, bei höheren Einsatzkonzentrationen aber sehr klebrig zu wirken.

Es war daher die Aufgabe der vorliegenden Erfindung, ein Hautpflegemittel auf der Basis von Glycerin zu entwickeln, dass weniger klebrig und stärker hautbefeuchtend ist.

Eine relativ neue Produktform zur Hautpflege stellen die unter der Dusche eingesetzten Hydrodispersionen dar, die nach einem ersten Duschvorgang auf die nasse Haut appliziert werden, wobei anschließend in einem zweiten Duschvorgang überschüssige, nicht auf der Haut fixierte Zubereitung abgespült wird (sogenannte In-shower Produkte, die analog wie Duschgele angewendet werden, im Gegensatz zu diesen jedoch nicht reinigen, sondern das Pflegeprodukt auf der Haut applizieren). Diese Produkte haben häufig den Nachteil, das die durch sie hervorgerufene Hautbefeuchtung durch das Produkt selbst nur unzureichend ist und nicht lange genug anhält.

Es war daher die Aufgabe der vorliegenden Erfindung, ein In-shower Produkt zu entwickeln, welches stärker und länger Hautbefeuchtend wirkt als die Zubereitungen des Standes der Technik.

Nachteilig an den herkömmlichen In-Shower Produkten ist nicht zuletzt der Umstand, dass sich mit ihnen kaum UV-Lichtschutzfilter für den UV-Schutz der Haut wirksam applizieren lassen. Meist werden die UV-Filter beim zweiten Duschvorgang mehr oder weniger vollständig von der Haut wieder abgespült, so dass praktisch kein UV-Schutz entstehen kann.

Es war daher die Aufgabe der vorliegenden Erfindung, ein In-Shower Produkt zu entwickeln, mit dem sich größeren Mengen an UV-Filtern wirksam auf die Haut applizieren lassen. Insbesondere sollten Lichtschutzfaktoren von mindestens SPF 6 bei einer normalen Anwendung erreichen lassen.

Überraschend gelöst werden die Aufgaben durch eine kosmetische Zubereitung nach einem der Ansprüche 1 und 2.

Zwar kennt der Stand der Technik Anonymous: "Discover the latest ingredinets, formulations and the most innovative beauty products" INNOVATION ZONE 2012, IN-COSMETICS ASIA, 1.November 2012, WO 2009/135755, WO 03/059311 und WO 2013/064391, doch konnten diese Schriften nicht den Weg zur vorliegenden Erfindung weisen.

Die erfindungsgemäße Glasübergangstemperatur wird dabei mit Hilfe der Differential Scanning Calorimetry bestimmt. Im Rahmen der vorliegenden Erfindung (d.h. erfindungsgemäß) wurde ein Mettler DCS 1 Gerät im Temperaturzyklus-Modus mit einem gelochten Aluminium-Tiegel (40µl), bei einer Messtemperatur von -50 °C bis +150 °C, einer Heizrate von 10 °C/min. und Stickstoff als Spülgas (Stickstoff 5.0) verwendet.

Die Hautbefeuchtung wird erfindungsgemäß mit dem folgenden Verfahren bestimmt: Zunächst wird in einer 1. Messung die Hautfeuchte mit dem Corneometer C 825 (Firma Courage & Khazaka) an 4 Arealen an den Unterarmen bestimmt. Dieser Wert entspricht der Hautfeuchte unbehandelter Haut.

Anschließend werden die vermessenen Areale an den Unterarmen wie folgt vorkonditioniert: Sie werden 10s unter laufendem Wasser, ca. 32-35°C warm, gehalten, dann mit 2mg/cm² Duschbad ,10s einschäumt und unter laufendem Wasser 15s abgespült.

Daraufhin werden die nasse Areale 10s mit der zu testenden Zubereitung in einer Auftragungsmenge von 2mg/cm² eincremt, 15s mit 32-35°C warmen Wasser abwaschen und mit einem Handtusch trocken getupft und die Hautfeuchte mit dem Corneometer C 825 gemessen. Die Messung wir nach 1h wiederholt.

Es ist erfindungsgemäß vorteilhaft, wenn die Zubereitung das Acrylat-Copolymer in einer Konzentration von 0,1 bis 10 Gewichts-%, bezogen auf das Gesamtgewicht der Zubereitung, enthält.

Es ist erfindungsgemäß bevorzugt, wenn die Zubereitung das Acrylat-Copolymer in einer Konzentration von 0,5 bis 5 Gewichts-%, bezogen auf das Gesamtgewicht der Zubereitung, enthält.
Erfindungsgemäß vorteilhafte Ausführungsformen der vorliegenden Erfindung sind dadurch gekennzeichnet, dass die Zubereitung Glycerin in einer Konzentration von 0,1 bis 20% Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung Glycerin in einer Konzentration von 0,5 bis 15 Gewichts-%, bezogen auf das Gesamtgewicht der Zubereitung, enthält.

Es ist vorteilhaft im Sinne der vorliegenden Erfindung, wenn das Acrylat-Copolymer einen pH-Wert von 4,5 bis 6,5 aufweist.
Das erfindungsgemäße Acrylat-Copolymer kann beispielsweise unter dem Handelsnamen Epitex 66 Polymer bei der Firma Dow erworben werden.

Es ist erfindungsgemäß vorteilhaft, wenn die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß bevorzugt, wenn als UV-Filter eine oder mehrere Verbindungen gewählt aus der Gruppe 4-(tert.-Butyl)-4'-methoxydibenzoylmethan, 2-Phenylbenzimidazol-5-sulfonsäure und/oder deren Salze, Ethylhexylsalicylat, Homomenthylsalicylat, 2-Ethylhexyl-2-cyano-3,3-diphenylacrylat, 4,4',4"-(1,3,5-Triazin-2,4,6-triyltriimino)-tris-benzoësäure-tris(2-ethylhexylester) und 2,4-Bis-{[4-(2-ethyl-hexyloxy)-2-hydroxy]-phenyl}-6-(4-methoxyphenyl)-1,3,5-triazin gewählt werden.

Erfindungsgemäß vorteilhafte Ausführungsformen sind außerdem dadurch gekennzeichnet, dass die Zubereitung frei ist von Propyl- und Butylparaben, 3-lod-2-propinylbutylcarbamat, Methylisothiazolinon, 3-(4-Methylbenzyliden)-campher und 2-Hydroxy-4-methoxybenzophenon (Oxybenzon).

Hingegen kommt man zu erfindungsgemäß vorteilhaften Ausführungsformen, wenn die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Außerdem ist es erfindungsgemäß vorteilhaft, wenn die Zubereitung Phenoxyethanol und/oder Methylparaben enthält.

Es ist vorteilhaft im Sinne der vorliegenden Erfindung, wenn die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Erfindungsgemäß vorteilhaft ist es auch, wenn die erfindungsgemäße Zubereitung einen oder mehrere Komplexbildner enthält. Erfindungsgemäß bevorzugt ist dabei der Einsatz von Ethylendiamintetraessigsäure-Alkalisalzen (EDTA).

Erfindungsgemäß bevorzugt enthält die erfindungsgemäße Zubereitung von 0,02 bis 1,5% Gewichts-% EDTA, bezogen auf das Gesamtgewicht der Zubereitung.

Erfindungsgemäß vorteilhaft ist es auch, wenn die Zubereitung einen oder mehrere Emulgatoren gewählt aus der Gruppe der Verbindungen Glycerylstearatcitrat, Cetearylalkohol, Natriumcetearylsulfat + Glycerylstearat, Cetearylsulfosuccinat Natriumstearoylglutamat, Polyglyceryl-3-methylglucosedistearat,
Stearinsäure, Kaliumcetylphosphat, enthält. Dabei ist ein Gehalt von Polyglyceryl-3-methylglucosedistearat erfindungsgemäß bevorzugt.

Enthält die erfindungsgemäße Zubereitung Polyglyceryl-3-methylglucosedistearat, so wird dieser Emulgator in einer Konzentration von 0,05 bis 5 Gewichts-%, bezogen auf das Gesamtgewicht der Zubereitung eingesetzt.

Es ist erfindungsgemäß von Vorteil, wenn die erfindungsgemäße Zubereitung Hydroxyethylcellulose und/oder Acrylat/C10-30 Alkylacrylat Crosspolymer enthält.

Enthält die Zubereitung Hydroxyethylcellulose, so ist es erfindungsgemäß bevorzugt, wenn diese Verbindung in einer Konzentration von 0,05 bis 5% Gewichts-%, bezogen auf das Gesamtgewicht der Zubereitung eingesetzt wird.

Enthält die Zubereitung Acrylat/C10-30 Alkylacrylat Crosspolymer, so ist es erfindungsgemäß bevorzugt, wenn diese Verbindung in einer Konzentration von 0,05 bis 2,5 Gewichts-%, bezogen auf das Gesamtgewicht der Zubereitung eingesetzt wird.

Erfindungsgemäß vorteilhafte Ausführungsformen sind auch dadurch gekennzeichnet, dass die Zubereitung Ethanol enthält.

Enthält die Zubereitung Ethanol, so ist es erfindungsgemäß bevorzugt, wenn diese Verbindung in einer Konzentration von 1 bis 15 Gewichts-%, bezogen auf das Gesamtgewicht der Zubereitung eingesetzt wird.

Die Ölphase der erfindungsgemäßen Zubereitung kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß bevorzugt ist es, wenn die erfindungsgemäße Zubereitung Dialkylcarbonat, Dialkyladipat und/oder Dialkylglutarat enthält.

Erfindungsgemäß besonders bevorzugt ist es, wenn die erfindungsgemäße Zubereitung Din-Octylcarbonat (INCI Dicaprylyl Carbonate) und/oder Di-n-Butyladipat (INCI Dibutyl Adipate) enthält.

Die erfindungsgemäße Zubereitung kann darüber hinaus die üblichen Inhaltstoffe enthalten und wie eine übliche Zubereitung zusammengesetzt sein.

Die erfindungsgemäße Zubereitung kann insbesondere vorteilhaft als Tagespflegeprodukt oder Sonnenschutzmittel eingesetzt werden.

Erfindungsgemäß besonders bevorzugt ist es, die erfindungsgemäße Zubereitung als In-Shower Produkt einzusetzen. Daher sind In-Shower Produkte auf der Basis einer erfindungsgemäßen kosmetischen Zubereitung erfindungsgemäß.

Erfindungsgemäß ist auch die Verwendung der erfindungsgemäßen kosmetischen Zubereitung als In-Shower Produkt.

Enthält die erfindungsgemäße Zubereitung einen oder mehrere UV-Filter, so ist die Verwendung dieser erfindungsgemäßen Zubereitung zum Schutz der Haut vor UV-Strahlung erfindungsgemäß.
Erfindungsgemäß ist auch das Verfahren zur Befeuchtung und Pflege der Haut, welches dadurch gekennzeichnet ist, dass die erfindungsgemäße Zubereitung nach dem Duschen auf die nasse Haut aufgetragen wird und anschließend überschüssige Reste der Zubereitung mittels einer Wasserdusche wieder abgespült werden.

Erfindungsgemäß ist ferner das Verfahren zum Schutz der Haut vor UV-Strahlung, welches dadurch gekennzeichnet ist, dass eine erfindungsgemäße Zubereitung, die einen oder mehrere der erfindungsgemäß vorteilhaften UV-Filter enthält, nach dem Duschen auf die nasse Haut aufgetragen wird und anschließend überschüssige Reste der Zubereitung mittels einer Wasserdusche wieder abgespült werden.

Erfindungsgemäß bevorzugt schließt sich bei beiden Verfahren als weiterer Verfahrensschritt das Abtrocknen der Haut (bevorzugt mittels eines Handtuches) an.

### Vergleichsversuch

Mit dem folgenden Versuch konnte der erfindungsgemäße Effekt beispielhaft belegt werden: Es wurden die folgenden Rezepturen hergestellt:

| | **1** | **2** | **3** |
|---|---|---|---|
| **INCI** | **m [%]** | **m [%]** | **m [%]** |
| Epitex 66 Acrylates Copolymer¹ | 2,00 | | |
| Vergleichspolymer Acrylates Copolymer² | | 2,00 | |
| Polyglyceryl-3 Methylglucose Distearate | 0,10 | 0,10 | 0,10 |
| Parfum | 0,40 | 0,40 | 0,40 |
| Glycerin + Aqua | 1,00 | 1,00 | 1,00 |
| Aqua + Sodium Hydroxide | 0,30 | 0,30 | 0,30 |
| Phenoxyethanol | 0,50 | 0,50 | 0,50 |
| Hydroxyethylcellulose | 0,10 | 0,10 | 0,10 |
| Acrylates/C10-30 Alkyl Acrylate Crosspolymer | 0,20 | 0,20 | 0,20 |
| Carbomer | 0,10 | 0,10 | 0,10 |
| Aqua | 52,27 | 50,47 | 56,27 |
| Alcohol Denat. + Aqua | 10,00 | 10,00 | 10,00 |
| Trisodium EDTA | 1,00 | 1,00 | 1,00 |
| Butyl Methoxydibenzoylmethane | 3,50 | 3,50 | 3,50 |
| Phenylbenzimidazole Sulfonic Acid | 0,10 | 0,10 | 0,10 |
| Ethylhexyl Salicylate | 4,50 | 4,50 | 4,50 |
| Ethylhexyl Triazone | 1,00 | 1,00 | 1,00 |
| Octocrylene | 9,00 | 9,00 | 9,00 |
| Homosalate | 9,00 | 9,00 | 9,00 |
| Bis-Ethylhexyloxyphenol Methoxyphenyl Triazine | 1,00 | 1,00 | 1,00 |
| Diethylhexyl Butamido Triazone | 0,50 | 0,50 | 0,50 |
| Diethylamino Hydroxybenzoyl Hexyl Benzoate | 1,00 | 1,00 | 1,00 |
| Titanium Dioxide (nano) + Silica + Methicone + Aqua | 0,43 | 0,43 | 0,43 |

| | | | |
|---|---|---|---|
| ¹Einsatzmenge Epitex 66=4,4 Gew-%. Diese entspricht dann der angegebenen Acrylates Crosspolymer Konzentration von 2 Gewichts-% (effektiv Wert) ² Daitosol 3000SLPN von Daitosakai mit einer Glasübergangstemperatur von + 14,5 | | | |

Von diesen Zubereitungen wurde die Hautbefeuchtungsleistung mit dem folgenden Verfahren bestimmt:
Zunächst wird in einer 1. Messung die Hautfeuchte mit dem Corneometer C 825 (Firma Courage & Khazaka) an 4 Arealen an den Unterarmen bestimmt. Dieser Wert entspricht der Hautfeuchte unbehandelter Haut.

Anschließend werden die vermessenen Areale an den Unterarmen wie folgt vorkonditioniert: Sie werden 10s unter laufendem Wasser, ca. 32-35°C warm, gehalten, dann mit 2mg/cm² Duschbad ,10s einschäumt und unter laufendem Wasser 15s abgespült.

Daraufhin werden die nasse Areale 10s mit der zu testenden Zubereitung in einer Auftragungsmenge von 2mg/cm2 eincremt, 15s mit 32-35°C warmen Wasser abwaschen und mit einem Handtusch trocken getupft und die Hautfeuchte mit dem Corneometer C 825 gemessen. Die Messung wird nach 1h wiederholt.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **5** | **6** | **7** | **8** |
|---|---|---|---|---|
| **INCI** | **m [%]** | **m [%]** | **m [%]** | **m [%]** |
| Epitex 66 (Acrylates Crosspolymer)¹ | 2,00 | 2,00 | 2,00 | 2,00 |
| Polyglyceryl-3 Methylglucose Distearate | 0,10 | 0,20 | 0,30 | 0,50 |
| Parfum | 0,40 | 0,40 | 0,40 | 0,40 |
| Glycerin + Aqua | 5,00 | 2,00 | 1,50 | 9,00 |
| Aqua + Sodium Hydroxide | 0,30 | 0,40 | 0,40 | 0,50 |
| Phenoxyethanol | 0,50 | 0,50 | 0,60 | 0,50 |
| Hydroxyethylcellulose | 0,10 | 0,30 | 0,30 | 0,20 |
| Acrylates/C10-30 Alkyl Acrylate Crosspolymer | 0,20 | 0,10 | 0,30 | 0,20 |
| Carbomer | 0,20 | 0,30 | 0,10 | 0,30 |
| Alcohol Denat. + Aqua | 10,00 | 8,00 | 5,00 | 10,00 |
| Trisodium EDTA | 1,00 | 1,00 | 1,00 | 1,00 |
| Paraffinum Liquidum | 2,00 | 2,00 | 2,00 | 2,00 |
| Caprylic/Capric Triglyceride | 3,00 | 3,00 | 2,00 | 3,00 |
| Isopropyl Palmitate | 2,00 | 1,00 | 3,00 | 1,00 |
| Dicaprylyl Ether | 3,00 | 2,00 | 1,00 | 2,00 |
| C13-16 Isoparaffin | 3,00 | 4,00 | 5,00 | 3,00 |
| Dimethicone | 1,00 | 1,00 | 2,00 | 1,50 |
| Aqua | ad 100 | ad 100 | ad 100 | ad 100 |

| | | | | |
|---|---|---|---|---|
| ¹Einsatzmenge Epitex 66=4,4 Gew-%. Diese entspricht dann der angegebenen Acrylates Crosspolymer Konzentration von 2 Gewichts-% (effektiv Wert) | | | | |

| | **9** | **10** | **11** | **12** |
|---|---|---|---|---|
| **INCI** | **m [%]** | **m [%]** | **m [%]** | **m [%]** |
| Epitex 66 (Acrylates Crosspolymer)¹ | 2,0 | 3,0 | 5,0 | 4,0 |
| Polyglyceryl-3 Methylglucose Distearate | 0,1 | 0,1 | 0,1 | 0,1 |
| Parfum | 0,4 | 0,4 | 0,4 | 0,4 |
| Glycerin + Aqua | 9,0 | 5,0 | 5,0 | 4,6 |
| Aqua + Sodium Hydroxide | 0,3 | 0,3 | 0,3 | 0,3 |
| Phenoxyethanol | 0,5 | 0,5 | 0,5 | 0,5 |
| Hydroxyethylcellulose | 0,1 | 0,1 | 0,1 | 0,1 |
| Acrylates/C10-30 Alkyl Acrylate Crosspolymer | 0,2 | 0,2 | 0,2 | 0,2 |
| Carbomer | 0,1 | 0,1 | 0,1 | 0,1 |
| Alcohol Denat. + Aqua | 10,0 | 10,0 | 10,0 | 10,0 |
| Trisodium EDTA | 1,0 | 1,0 | 1,0 | 1,0 |
| Butyl Methoxydibenzoylmethane | 2,5 | 3,5 | 4,3 | 4,9 |
| Ethylhexyl Salicylate | 4,5 | | | 4,5 |
| Ethylhexyl Triazone | 1,0 | 1,0 | | 1,0 |
| Octocrylene | 2,3 | 9,0 | 5,0 | 9,0 |
| Homosalate | 9,0 | | 9,0 | 9,0 |
| Bis-Ethylhexyloxyphenol Methoxyphenyl Triazine | 1,0 | 1,0 | 1,0 | 1,0 |
| Diethylhexyl Butamido Triazone | 0,5 | 0,5 | 0,5 | 0,5 |
| Diethylamino Hydroxybenzoyl Hexyl Benzoate | 1,0 | 1,0 | 1,0 | 1,0 |
| Phenylbenzimidazole Sulfonic Acid | 1,0 | 2,0 | | |
| Disodium Phenyl Dibenzimidazole Tetrasulfonate | 0,2 | 0,5 | | |
| Methylene Bis-Benzotriazolyl Tetra methyl butylphenol | | | 3,0 | 4,4 |
| Tris-Biphenyl Triazine | | | 1,0 | 3,0 |
| Titanium Dioxide | | | 3,0 | 3,2 |
| Aqua | ad 100 | ad 100 | ad 100 | ad 100 |

| | | | | |
|---|---|---|---|---|
| ¹Einsatzmenge Epitex 66=4,4 Gew-%. Diese entspricht dann der angegebenen Acrylates Crosspolymer Konzentration von 2 Gewichts-% (effektiv Wert) | | | | |

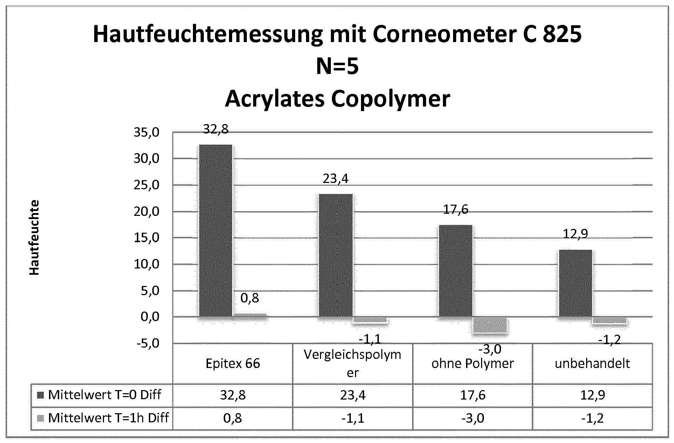

## Patentansprüche

1. Kosmetische Zubereitung enthaltend
a) ein Acrylat-Copolymer mit einer Glasübergangstemperatur von -5 bis -15 C°, gemessen mittels DSC und
b) Glycerin,
die frei ist von partikulären Eisenoxiden,
**dadurch gekennzeichnet, dass** die Zubereitung Hydroxyethylcellulose und/oder Acrylat/C10-30 Alkylacrylat Crosspolymer enthält.

2. Kosmetische Zubereitung enthaltend
a) ein Acrylat-Copolymer mit einer Glasübergangstemperatur von -5 bis -15 C°, gemessen mittels DSC und
b) Glycerin,
die frei ist von partikulären Eisenoxiden,
**dadurch gekennzeichnet, dass** die Zubereitung frei ist von Propyl- und Butylparaben, 3-lod-2-propinylbutylcarbamat, Methylisothiazolinon, 3-(4-Methylbenzyliden)-campher und 2-Hydroxy-4-methoxybenzophenon (Oxybenzon).

3. Kosmetische Zubereitung nach Anspruch 1 oder 2, **dadurch gekennzeichnet, dass** die Zubereitung das Acrylat-Copolymer in einer Konzentration von 0,3 bis 5 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Glycerin in einer Konzentration von 0,5 bis 15 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethy)-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

6. Kosmetische Zubereitung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

7. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

8. Kosmetische Zubereitung nach einem der vorhergehenden Ansprüche, **dadurch gekennzeichnet, dass** die Zubereitung Phenoxyethanol und/oder Methylparaben enthält.

9. Kosmetische Zubereitung nach einem der vorhergehenden Ansprüche, **dadurch gekennzeichnet, dass** die Zubereitung einen oder mehrere Komplexbildner enthält.

10. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat, Stearinsäure, Kaliumcetylphosphat, enthält.

11. Kosmetische Zubereitung nach einem der vorhergehenden Ansprüche, **dadurch gekennzeichnet, dass** die Zubereitung Ethanol enthält.

12. In-shower Produkt auf der Basis einer kosmetischen Zubereitung nach einem der Ansprüche 1 bis 11.

13. Verwendung einer kosmetischen Zubereitung nach einem der vorhergehenden Ansprüche als In-shower Produkt.

14. Verfahren zur Befeuchtung und Pflege der Haut, **dadurch gekennzeichnet, dass** eine Zubereitung nach Anspruch 1 bis 11 nach dem Duschen auf die nasse Haut aufgetragen wird und anschließend überschüssige Reste der Zubereitung mittels einer Wasserdusche wieder abgespült werden.

## Claims

1. Cosmetic preparation comprising
a) an acrylate copolymer having a glass transition temperature of -5 to -15°C, measured by DSC and
b) glycerin,
which is free from particulate iron oxides,
**characterized in that** the preparation comprises hydroxyethylcellulose and/or acrylate/C10-30 alkyl acrylate cross polymer.

2. Cosmetic preparation comprising
a) an acrylate copolymer having a glass transition temperature of -5 to -15°C, measured by DSC and
b) glycerin,
which is free from particulate iron oxides,
**characterized in that** the preparation is free from propylparaben and butylparaben, 3-iodo-2-propynyl butylcarbamate, methylisothiazolinone, 3-(4-methylbenzylidene)camphor and 2-hydroxy-4-methoxybenzophenone (oxybenzone).

3. Cosmetic preparation according to Claim 1 or 2, **characterized in that** the preparation comprises the acrylate copolymer at a concentration of 0.3 to 5% by weight, based on the total weight of the preparation.

4. Cosmetic preparation according to any of the preceding claims, **characterized in that** the preparation comprises glycerin at a concentration of 0.5 to 15% by weight, based on the total weight of the preparation.

5. Cosmetic preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane-copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

6. Cosmetic preparation according to any of the preceding claims, **characterized in that** the preparation comprises ethylhexylglycerin, propylene glycol, butylene glycol, 2-methylpropane-1,3-diol, 1,2-pentanediol, 1,2-hexanediol, 1,2-octanediol and/or 1,2-decanediol.

7. Cosmetic preparation according to any of the preceding claims, **characterized in that** the preparation comprises one or more active ingredients selected from the group of compounds comprising magnolia extract, gylcyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

8. Cosmetic preparation according to any of the preceding claims, **characterized in that** the preparation comprises phenoxyethanol and/or methylparaben.

9. Cosmetic preparation according to any of the preceding claims, **characterized in that** the preparation comprises one or more chelating agents.

10. Cosmetic preparation according to any of the preceding claims, **characterized in that** the preparation comprises one or more emulsifiers selected from the group of compounds comprising glyceryl stearate citrate, cetearyl alcohol, sodium cetearyl sulfate + glyceryl stearate, cetearyl sulfosuccinate, sodium stearoyl glutamate, polyglyceryl-3 methylglucose distearate, stearic acid, potassium cetyl phosphate.

11. Cosmetic preparation according to any of the preceding claims, **characterized in that** the preparation comprises ethanol.

12. In-shower product based on a cosmetic preparation according to any of Claims 1 to 11.

13. Use of a cosmetic preparation according to any of the preceding claims as an in-shower product.

14. Method for moisturizing and caring for skin, **characterized in that** a preparation according to Claims 1 to 11 is applied to wet skin after showering and subsequently excess residues of the preparation are rinsed off again by means of a water shower.

## Revendications

1. Préparation cosmétique contenant
a) un copolymère d'acrylate présentant une température de transition vitreuse de -5 à -15°C, mesurée par DSC, et
b) du glycérol,
qui est exempte d'oxydes de fer particulaires, **caractérisée en ce que** la préparation contient de l'hydroxyéthylcellulose et/ou un copolymère réticulé d'acrylate/acrylate de C10-30-alkyle.

2. Préparation cosmétique contenant
a) un copolymère d'acrylate présentant une température de transition vitreuse de -5 à -15°C, mesurée par DSC, et
b) du glycérol,
qui est exempte d'oxydes de fer particulaires, **caractérisée en ce que** la préparation est exempte de propylparabène ainsi que de butylparabène, de butylcarbamate de 3-iodo-2-propynyle, de méthylisothiazolinone, de 3-(4-méthylbenzylidène)-camphre et de 2-hydroxy-4-méthoxybenzophénone (oxybenzone).

3. Préparation cosmétique selon la revendication 1 ou 2, **caractérisée en ce que** la préparation contient le copolymère d'acrylate en une concentration de 0,3 à 5% en poids par rapport au poids total de la préparation.

4. Préparation cosmétique selon l'une quelconque des revendications précédentes, **caractérisée en ce que** la préparation contient le glycérol en une concentration de 0,5 à 15% en poids par rapport au poids total de la préparation.

5. Préparation cosmétique selon l'une quelconque des revendications précédentes, **caractérisée en ce que** la préparation contient un ou plusieurs filtres UV, choisis dans le groupe formé par les composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1 - [(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; 2-cyano-3,3-diphénylacrylate de 2-éthylhexyle ; ester 2-éthylhexylique de l'acide 4-(diméthylamino)-benzoïque ; ester amylique de l'acide 4-(diméthylamino)benzoïque ; ester di-(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthylhexyle ; diméthicodiéthylbenzalmalonate ; copolymère de 3-(4-(2,2-bis-éthoxycarbonylvinyl)-phénoxy)propényl)-méthoxysiloxane/diméthylsiloxane ; 4-(tert-butyl)-4'-méthoxydibenzoylméthane ; ester hexylique de l'acide 2-(4'-diéthylamino-2'-hydroxybenzoyl)-benzoïque ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (présentant le n° CAS 288254-16-0) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanines ; dioxyde de titane ; oxyde de zinc.

6. Préparation cosmétique selon l'une quelconque des revendications précédentes, **caractérisée en ce que** la préparation contient de l'éthylhexylglycérol, du propylèneglycol, du butylèneglycol, du 2-méthylpropane-1,3-diol, du 1,2-pentanediol, du 1,2-hexanediol, du 1,2-octanediol et/ou du 1,2-décanediol.

7. Préparation cosmétique selon l'une quelconque des revendications précédentes, **caractérisée en ce que** la préparation contient une ou plusieurs substances actives choisies dans le groupe des composés extrait de magnolia, acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

8. Préparation cosmétique selon l'une quelconque des revendications précédentes, **caractérisée en ce que** la préparation contient du phénoxyéthanol et/ou du méthylparabène.

9. Préparation cosmétique selon l'une quelconque des revendications précédentes, **caractérisée en ce que** la préparation contient un ou plusieurs complexants.

10. Préparation cosmétique selon l'une quelconque des revendications précédentes, **caractérisée en ce que** la préparation contient un ou plusieurs émulsifiants, choisis dans le groupe des composés citrate-stéarate de glycéryle, alcool cétéarylique, cétéarylsulfate de sodium + stéarate de glycéryle, sulfosuccinate de cétéaryle, stéaroylglutamate de sodium, distéarate de polyglycéryl-3-méthylglucose, acide stéarique, cétylphosphate de potassium.

11. Préparation cosmétique selon l'une quelconque des revendications précédentes, **caractérisée en ce que** la préparation contient de l'éthanol.

12. Produit à utiliser sous la douche à base de la préparation cosmétique selon l'une quelconque des revendications 1 à 11.

13. Utilisation d'une préparation cosmétique selon l'une quelconque des revendications précédentes en tant que produit à utiliser sous la douche.

14. Procédé d'hydratation et de soin de la peau, **caractérisé en ce qu'**une préparation selon les revendications 1 à 11 est appliquée après la douche sur la peau humide et les restes en excès de la préparation sont ensuite éliminés par rinçage au moyen d'une douche à eau.
